# EUROPEAN PATENT SPECIFICATION

(11) **EP 3 238 193 B1**
(45) Date of publication and mention of the grant of the patent: **08.04.2020**
(21) Application number: 15820131.9
(22) Date of filing: 21.12.2015
(51) Int. Cl.: G08B 21/02, G08B 21/04, A61B 5/00, A61B 5/024

(54) **FIRST AID KIT WEARABLE**
TRAGBARES ERSTES-HILFE-KIT
KIT DE PREMIERS SOINS PORTABLE

(30) Priority: 22.12.2014 US 201462095234 P; 11.06.2015 EP 15171603
(43) Date of publication of application: 01.11.2017
(73) Proprietor: Koninklijke Philips N.V., 5656 AG Eindhoven (NL)
(72) Inventor: CRONIN, John, 5656 AE Eindhoven (NL); BODKIN, Joseph George, 5656 AE Eindhoven (NL)
(74) Representative: de Haan, Poul Erik
(86) International application number: PCT/EP2015/080751
(87) International publication number: WO 2016/102439

(56) References cited:
- WO-A1-2013/038230
- WO-A2-2006/001005
- DE-A1-102011 103 339
- JP-A- 2006 259 939
- US-A1- 2012 044 069

## Description

### CROSS-REFERENCE TO RELATED APPLICATIONS

The present application claims the priority benefit of U.S. provisional application number 62/095,234 filed December 22, 2014 and entitled "First Aid Kit Wearable.

### TECHNICAL FIELD

Various embodiments described herein generally relate to wearable devices and first aid alert systems. More specifically, but not exclusively, the wearable device sends health related data to a first aid server and the first aid server transmits a recommendation that includes first aid instructions or that initiates an emergency call automatically.

### BACKGROUND

Wearable technology includes mobile electronic devices that may be worn on the body or attached to or embedded in clothes and accessories of an individual. The designs of wearable technology often incorporate practical functions and features, but may also have a purely critical or aesthetic agenda. Processors and sensors associated with the wearable technology may gather, process, and display information to a user. Wearable technology may be utilized in a variety of areas including monitoring health data of a user and providing other types of data and statistics. Examples of wearable technology in the health arena include the FITBIT, the NIKE+ FUELBAND, and APPLE WATCH devices.

Presently available electronic sensors coupled to a computing device are used to collect and manipulate data sensed by the electronic sensors. Sensors that sense acceleration are used to collect data relating to motions of a person wearing a wearable electronic device. For example, certain devices have accelerometer sensors that count a number of steps by measuring acceleration in three dimensions (X, Y, and Z) during an activity (*e.g*., walking). Some wearable devices allow the user operating a GUI in an app or web app to enter other data related to primary parameters (*e.g.,* number of calories consumed). In such devices, sensor data and user input may be stored in memory, and a processor running an algorithm may identify patterns in the data that corresponds to a series of sensations sensed by the sensors. Wearable computing devices coupled to sensors may also be used to sense the activity of a person wearing the computing device when exercising. A wearable device may also make measure physiological parameters of a person. Examples of physiological parameters that may be measured include heart rate and steps walked/ran. In some cases, such products may determine whether a person is running or walking based on data sensed by the sensors. This may be done based on counting steps, where the data is sensed by an acceleration sensor over a period of time. Typical wearable devices gather sensor measurements and keep these measurements within internal memory. Thus, it may be cumbersome and unwieldy to move this sensor data, or to perform actions based on situations involving particular measurements produced by the sensors.

US20120044069A1 discloses a wellbeing transponder system. The described system and method shall enable user health monitoring and alerting by providing an arm-mounted sensor wirelessly linked to a wrist-mounted transponder, although the wrist-mounted transponder may be omitted in favor of a user-carried cellular device. A remote server is included to receive user health data and alerts, and to log received data and, when necessary, to alert health service providers. Health service providers may include physicians and emergency services providers.

### SUMMARY

Various embodiments described herein relate to a first-aid kit and related method and non-transitory machine-readable media including: at least one first aid item; and a wearable device configured to be worn by a user and including: a communications interface for communicating with a mobile device of the user; a sensor for obtaining vitals data when the wearable device is worn by the user; a processor configured to transmit vitals data to a mobile phone of the user via the communications interface. The at least one first aid item includes an identifier and the processor of the wearable device is further configured to: read the identifier from the at least one first aid item; and (a) determine, based at least in part of reading the identifier, that the at least one first aid item is in use; and transmit, to the mobile device, an indication that the at least one first aid item is in use; or (b) transmit the identifier to the mobile device via the communication interface.

Various embodiments described herein relate to a wearable device for inclusion in a first aid kit and related method and non-transitory machine-readable media including: a communications interface for communicating with a mobile device of a user; a sensor for obtaining vitals data when the wearable device is worn by the user; a processor configured to transmit vitals data and an identification of the at least one first aid kit item to a mobile phone of the user via the communications interface. The at least one first aid item includes an identifier and the processor of the wearable device is further configured to: read the identifier from the at least one first aid item; and (a) determine, based at least in part of reading the identifier, that the at least one first aid item is in use; and transmit, to the mobile device, an indication that the at least one first aid item is in use; or (b) transmit the identifier to the mobile device via the communication interface.

Various embodiments are described wherein the means for causing instructions for processing the vitals data to be loaded onto the mobile device includes: a memory storing contents including at least one of: the instructions for processing the vitals, and a resource identifier that points to the instructions for processing the vitals at an application download server, wherein the contents of the memory are transmitted to the mobile device.

Various embodiments are described wherein the wearable device includes the memory.

Various embodiments are described wherein the means for causing instructions for processing the vitals data to be loaded onto the mobile device includes: a visible indicia that, when read by a camera of the mobile device, communicates a resource identifier that points to the instructions for processing the vitals at an application download server.

Various embodiments are described wherein, in reading the identifier, the processor is configured to read the identifier via short-range wireless communication.

Various embodiments are described wherein the processor is further configured to transmit to the mobile device an identification of contents within the first-aid kit.

### BRIEF DESCRIPTION OF THE DRAWINGS

In order to better understand various example embodiments, reference is made to the accompanying drawings, wherein:
FIGURE 1 illustrates a wearable device from an example first aid kit that may communicate with a mobile electronic device or with a first aid server;
FIGURE 2A illustrates an example first aid kit graphical user interface (GUI) of the mobile device;
FIGURE 2B is a flowchart illustrating an example method that may be performed by base instructions 126 at a wearable device;
FIGURE 2C is a flowchart illustrating an example method performed by an alert system at the wearable device;
FIGURE 3A illustrates an example first aid kit graphical user interface (GUI) of the mobile device;
FIGURE 3B is a flowchart illustrating example operations of the base instructions of the wearable device as used by an emergency responder;
FIGURE 4 is a flowchart illustrating an example methodology that may be performed by base instructions in a mobile device first aid application (App);
FIGURE 5 is a flowchart illustrating example operations of an emergency dispatch instructions of a first aid server;
FIGURE 6 illustrates a mobile device architecture that may be utilized to implement the various features and processes described herein;
FIGURE 7A is a diagram of example injury-type selection boxes relating to first aid directions that may be selected at a first aid kit GUI of the mobile device;
FIGURE 7B is a flowchart illustrating an example process of generating first aid directions at a base instructions of the first aid server; and
FIGURE 8 is a flowchart illustrating an example methodology consistent with the example methods and principles described herein.

### DETAILED DESCRIPTION

It would be desirable to provide improved systems and methods for a wearable device to sense parametric health data and send that data to a first aid server that may determine whether emergency services, such as 911, should be called. Various embodiments described herein generally relate to a system and method for a wearable device collecting health related data and transmitting that data to a first aid server. The first aid server after receiving the health related data may transmit a signal that automatically initiates a mobile device to automatically call 911. The first aid server may also transmit first aid directions to a mobile device. The health related data may be sent from the wearable device to a mobile device and that data may be forwarded to the first aid server by the mobile device.

Wearable devices, a mobile device, and a first aid server may communicate using any data communication technology known in the art. In some cases, a wearable device may communicate with a mobile device using a first type of wireless data communication technology, and the mobile device may communicate with the first aid server using a second type of wireless data communication technology. Data communication interfaces useful in various embodiments include, yet are not limited, to cellular 3G-4G LTE, Wi-Fi (802.11), infrared, optical, near field, and Bluetooth data communication interfaces. In certain instances, a wearable device may include a plurality of data communication interfaces, a processor, a memory, a field programmable gate array (FPGA), an application specific integrated circuit (ASIC).

Mobile electronic devices described herein include, yet are not limited to smartphones, iPhones, Android phones, iPads, and notebook computers. Communications communicated by a wearable device or by a mobile device may be communicated over any data communication technology known in the art, including, yet not limited to Bluetooth, Cellular 3G 4G LTE, and Wi-Fi (802.11). In certain instances, a mobile device may include a plurality of data communication interfaces, a processor, a memory, a field programmable gate array (FPGA), an application specific integrated circuit (ASIC).

The various methods may be performed by software operating in conjunction with hardware. For example, instructions executed by a processor, the instructions otherwise stored in a non-transitory computer readable medium such as memory. Various interfaces may be implemented-both communications and interface. One skilled in the art will appreciate the various requisite components of a mobile device and integration of the same with one or more of the figures or descriptions included herein.

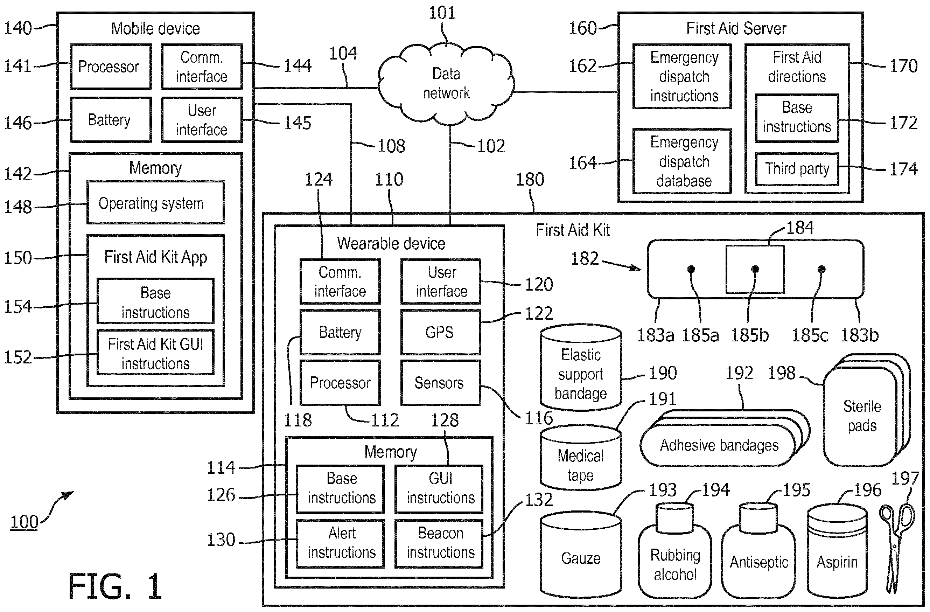

FIGURE 1 illustrates an example environment 100 including a wearable device 110 from an example first aid kit 180 that may communicate with a mobile electronic device 140 or with a first aid server 160. The wearable device 110 in FIGURE 1 includes a processor 112, one or more sensors 116, a memory 114, a battery 118, a user interface 120 (*e.g.,* a display, touchscreen, buttons, speaker, microphone, or other hardware for interacting with a user), a global positioning system (GPS) 122, a wired or wireless communications interface 124 (*e.g,* a USB port, a FireWire port, a Lightning port, a Thunderbolt port, a Wi-Fi connection, a 3G/4G/LTE cellular connection, a Bluetooth connection, a Bluetooth low energy connection, a Bluetooth Smart connection, a near field communication, a radio wave communications). The memory 114 may store instructions for execution by the processor 112 such as a base instructions 126, intelligent beacon instructions 132, alert instructions 130, and a graphical user interface instructions (GUI) 122. Examples of beacon instructions 132 include, yet are not limited to, instructions for sending a transmission sent over a wireless, optical, infrared, near field data communication transmission, and Bluetooth.

In various embodiments, the wearable device 110 may be a standalone device include within the kit 180 such as, for example, a bracelet, necklace, belt, or patch that includes one or more of the described components 112-132. In some embodiments, the one or more of the described components 112-132 may be integrated into one or more of the kit items 182-198. For example, the smart bandage 182, adhesive bandages 192, and elastic support bandages 190 may each constitute wearable devices 110.

As used herein, the term "processor" will be understood to include any hardware device capable of executing instructions stored in memory or otherwise processing data. As such, a processor may include a microprocessor, field programmable gate array (FPGA), application-specific integrated circuit (ASIC), or other similar devices. Accordingly, while various embodiments described herein explain various functions "performed" by instructions or software, it will be apparent that such functionality may, in fact, be performed by appropriate hardware such as a processor. In embodiments where some or all such described functionality is hard-wired into the construction of one or more ASICs, instructions defining such functionality may be omitted.

The term "memory" will be understood to include various memories such as, for example L1, L2, or L3 cache or system memory as well as storage memories such as flash, magnetic, optical storage media as well as other non-transitory storage media. As used herein, the term "non-transitory" will be understood to exclude transitory signals but to include all forms of storage, including both volatile and non-volatile memories.

In some embodiments, the wearable device 110 may have one sensor 116, while in other embodiments, the wearable device 110 may have multiple sensors 116. These sensors 116 may include, for example, sensors measuring hydration, calories, blood pressure, blood sugar or glucose, insulin, body temperature (*e.g.,* thermometer), heart rate, weight, sleep, number of steps (*e.g.,* pedometer), velocity or acceleration (*e.g.,* accelerometer), vitamin levels, respiratory rate, heart sound (*e.g.,* microphone), breathing sound (*e.g.,* microphone), movement speed, skin moisture, sweat detection, sweat composition, nerve firings (*e.g.,* electromagnetic sensor), or similar health measurements.

An example first aid kit 180 depicted in FIGURE 1 includes the wearable device 110, smart bandages 182, gauze193, rubbing alcohol 194, antiseptic 195, aspirin 196, sterile pads 198, elastic support bandages 190, medical tape 191, scissors 197, and adhesive bandages 192 of different sizes. It will be apparent that the contents of the first aid kit 180 depicted are merely examples and that some of these elements may be omitted or additional elements may be part of a first aid kit 180.

In some embodiments, some of the elements within the first aid kit 180 (*e.g.,* rubbing alcohol, antiseptic, bandages) may include an identifier (*e.g.,* a barcode, a serial number/code, a quick response (QR) code, a passive or active radio frequency identifier (RFID) tag, a near-field communication tag, a bluetooth connection, *etc.*)*.* In some embodiments, the wearable device 110, the mobile device 140, or the first aid server 160 may use these identifiers to track, poll, or otherwise note the current inventory of the first aid kit 180, and to note what elements of the first aid kit 180 are being used at a given time. This may be used to estimate when an emergency is underway (*e.g.,* antiseptic and bandages being removed from the first aid kit 180 likely means that an injury has occurred) so that the wearable device 110 or mobile device 140 may display advice (*e.g.,* if bandages have been removed the wearable device 110 may display the message: "use antiseptic on cuts to decrease chances of infection"), or ensure that the right elements or medications are being used (*e.g.,* if bandages and eyedrops have been removed, the wearable device 110 may display the message: "you are using eyedrops - did you mean to use antiseptic?"). To implement such functionality, one or more of the items in the first aid kit may include low-power wireless chip, such as an RFID chip, storing an identifier (*e.g.,* an identifier unique to the specific item or to the type of item) that may be read by hardware of the wearable device. Upon reading such an identifier, the wearable device may assume that the item is in use. For example, in some embodiments, the wearable device may assume that, if the item is close enough to be read, it must be in the hand of the user. Alternatively, the wearable device may determine that if the item is close enough to read but other items within the kit (*e.g.,* if only 3 items are within range compared to 20 tagged items in the kit) or a wireless chip associated with the kit container itself are not close enough to read, that the item within range was taken away from the kit and may be in use or intended for use. Various alternative methods for tracking the contents and usage status of items in the first aid kit will be apparent. For example, each kit item may include visible indicia that bear the identifier of the item which can be read by the wearable device 110 or mobile device 140 under operation of the first aid kit app.

In some embodiments, various types of first aid kits 180 may be available, where each type of first aid kit 180 may include specialized emergency items. For example, different items may be offered in a home first aid kit (*e.g.,* general injury and electrical emergency equipment), a fire first aid kit (*e.g.,* a fire extinguisher, gas mask, and fire blanket), an athletic first aid kit (*e.g.,* bandages, antiseptics, antibiotic ointments, and temporary casts), an electrician's first aid kit (*e.g.,* insulating gloves and facial shielding), a chemist's first aid kit (*e.g.,* fire extinguishers for chemical fires, eye rinse, and treatment ointments for chemical burns), a heart condition first aid kit (*e.g.,* blood pressure pills and a defibrillator), or an allergy first aid kit (*e.g.,* allergy pills, ointments, eyedrops, nasal sprays, and an epinephrine auto-injector). In some embodiments, the wearable device 110 may be customized (in hardware or software) based on the type of first aid kit 180 it is packed with. For example, a wearable device 110 of an athletic first aid kit 180 may include pulse sensors and display calories burned or bandaging advice, while a wearable device 110 of a fire first aid kit 180 may include respiratory rate sensors or carbon monoxide sensors and display fire extinguishing tips based on type of fire.

The wearable device 110 may communicate with the mobile device 140 directly (*e.g.,* connection 108) or may communicate with the mobile device 140 or the first aid server 160 (*e.g.,* one or more servers of the first aid server 160) by connecting through the data network 101 (*e.g.,* connection 102, then followed by connection 104 to the mobile device 140 or connection 106 to the first aid server 160). Data network 101 may be virtually any device or group of devices capable of facilitating data communications including, for example, a local area network, mobile carrier network, cloud computing infrastructure, or the Internet. Such communications may be made using the communications interface 124 or beacon instructions 132. It should be understood that the components of wearable device 110 as illustrated in FIGURE 1 and described above are illustrative rather than limiting. A wearable device 110 may not include all of these components or may include additional components not listed herein.

The mobile device 140 in FIGURE 1 includes a memory 142, a communication interface 144 (*e.g,* a USB port, a FireWire port, a Lightning port, a Thunderbolt port, a Wi-Fi connection, a 3G/4G/LTE cellular connection, a Bluetooth connection, a Bluetooth low energy connection, a Bluetooth Smart connection, a near field communication, a radio wave communications), a battery 146, and a user interface 145. In various embodiments, the memory 142 may store an operating system 148 (*e.g.,* the GOOGLE ANDROID or APPLE IOS operating system) and a first aid kit app 150. The first aid kit app 150 may include base instructions 154 and a first aid kit graphical user interface (GUI) instructions 152. In some embodiments, the mobile device 140 may connect directly with the wearable device 110 (*e.g.,* through connection 108). In some embodiments, the mobile device 140 may connect to the data network 101 (*e.g..,* through connection 104) and then further connect to the wearable device 110 (*e.g.,* through connection 102) or to the first aid server 160 (*e.g.,* through connection 106). In some embodiments, the wearable device 110 may use the mobile device 140 as a proxy to connect to the first aid server 160, such as in embodiments where the communications interface 124 or beacon instructions 132 allows the wearable device 110 to connect directly to the mobile device 140 (*e.g.,* through connection 108) but not to the data network 101 (*e.g.,* through connection 102). It should be understood that the components of mobile device 140 as illustrated in FIGURE 1 and described above are illustrative rather than limiting. A mobile device 140 may not include all of these components or may include additional components not listed herein.

User mobile device 150 may be, for example, a smartphone, a tablet, a laptop computer, a desktop computer, a gaming console, a smart television, a home entertainment system, a second wearable device, or another computing device.

The first aid kit server 160 may include emergency dispatch instructions 162, a emergency dispatch database 164, and first aid directions 170, which may include base instructions 172 and third party instructions 174. A smart bandage illustrated in FIGURE 1 illustrates a band aid that includes one or more sensors 185a,b,c, a sterile pad 184, and adhesive strips 183a,b. In some embodiments, some of these elements may be omitted or additional elements may be included.

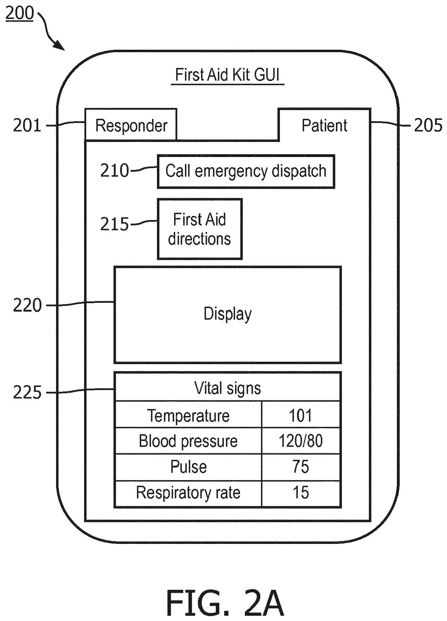

FIGURES 2A-C illustrate an example of the first aid kit graphical user interface (GUI) 200, a flowchart 229 illustrating example base instructions operations, and a flowchart 269 illustrating example alert system operations.

FIGURE 2A illustrates an example first aid kit graphical user interface (GUI) (*e.g.,* a GUI that may be created by the GUI instructions 152 of the mobile device 140 or GUI instructions 128 of the wearable device 120). The example first aid kit graphical user interface (GUI) 200 illustrates one embodiment of the first aid kit graphical user interface (GUI) 152 or GUI 128, but it should be noted that other embodiments of the first aid kit graphical user interface (GUI) 200 may operate differently. The example first aid kit GUI 200 of FIGURE 2A includes a responder tab 201 and a patient tab 205, where the patient tab 205 is selected. The first aid kit GUI 200 includes several selection buttons (*e.g.,* "call emergency dispatch" button 210, first aid directions buttons 215), a display box 220, and a set of vital signs data in a vital signs table 225. The selection buttons in the example first aid GUI 200 are emergency call selection button 210 and a first aid directions selection button 215. When the emergency dispatch button 210 is selected, a call may be placed to 911 or another emergency dispatch service. When the first aid directions selection button 215 is selected, a set of directions may be displayed in the first aid kit GUI 200 (*e.g.,* at the display 220) that may correspond to the set of vital signs data in the vital signs table 225. For example, directions that are displayed by the GUI 200 may also relate to how to dress a wound (*e.g.,* at the display 220). The vital signs table 225 illustrated in the first aid GUI 152 includes vital signs data regarding various sensor measurements of the wearable device 110 (*e.g.,* a temperature of 101 degrees F, blood pressure of 120/80, a pulse rate of 75, and a respiratory rate of 15).

The GUI elements of the example first aid kit GUI 200 of FIGURE 2A should be interpreted illustrative rather than limiting. Another embodiment of the first aid kit GUI 152 may not include all of these GUI elements or may include additional or different GUI elements not listed herein.

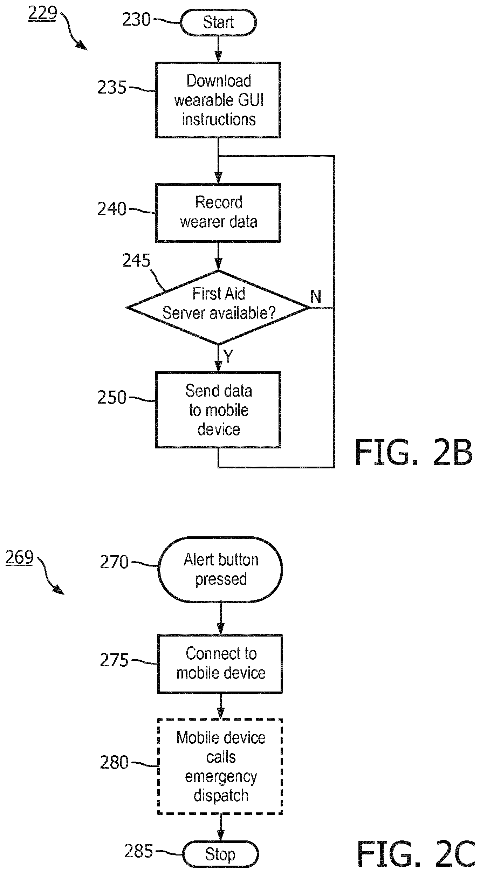

FIGURE 2B is a flowchart 229 illustrating an example method that may be performed by example base instructions 126 at a wearable device 110. Step 230 in the base instructions flowchart includes initiating the base instructions 126 by placing the wearable device 110 on a patient. In step 235, a wearable GUI 128 is downloaded or installed to the wearable device 110 through a beacon 132, if it has not been downloaded already. In step 240, the base instructions 126 in the wearable device 110 senses vital signs (*e.g.,* blood pressure, pulse), and then in step 245, the base instructions 126 determines whether a first aid server 160 is available (*e.g.,* whether it is accessible through the wearable device 110 or through a mobile device 140). When the first aid server 160 is determined not to be available in step 245, the base instructions 126 revert back to sensing vital signs in step 240 and checking up on the availability of the first aid server 160 until it is available in step 245. When the first aid server 160 is determined to be available in step 245, the base instructions 126 moves to step 250, which sends the vital signs to the mobile device (*e.g.,* directly through connection 108, or through the data network 101 through connections 102 and 104). In some embodiments, the vital signs data sensed at the wearable device 110 may also be sent to the first aid server 160 through the mobile device 140. In other instances vital signs data may be sent from the wearable device 110 directly to the first aid server 160 over the data network 101 without involving the mobile device 140. Different embodiments of the user base instructions 126 may perform different operations.

FIGURE 2C is a flowchart 269 illustrating an example method performed by an example alert instructions 130 at the wearable device 110. In step 270 of the alert system method flowchart, an alert button is pressed, for example, as a physical button on the wearable device or at the wearable device's GUI 128. While the flowchart 269 relates to a method performed by the wearable device, it will be apparent that similar instructions may be used by the mobile device, for example, upon a user pressing the emergency dispatch button 210 of the first aid kit GUI 200 of the mobile device 140. Next, in step 275, the wearable device 110 connects to the mobile device 140 using a beacon 132 or communications interface 124 (*e.g.,* using connection 108 or using the data network 101 through connections 102 and 104) and then the mobile device 140 calls 911 or another emergency number in step 280. Different embodiments of the alert instructions 130 may perform different operations. The method then proceeds to end in step 285.

While the flow diagrams in FIGURE 2B and FIGURE 2C show a particular order of operations performed by various embodiments, it should be understood that such order is an example (*e.g.,* alternative embodiments may perform the operations in a different order, combine certain operations, overlap certain operations, etc.).

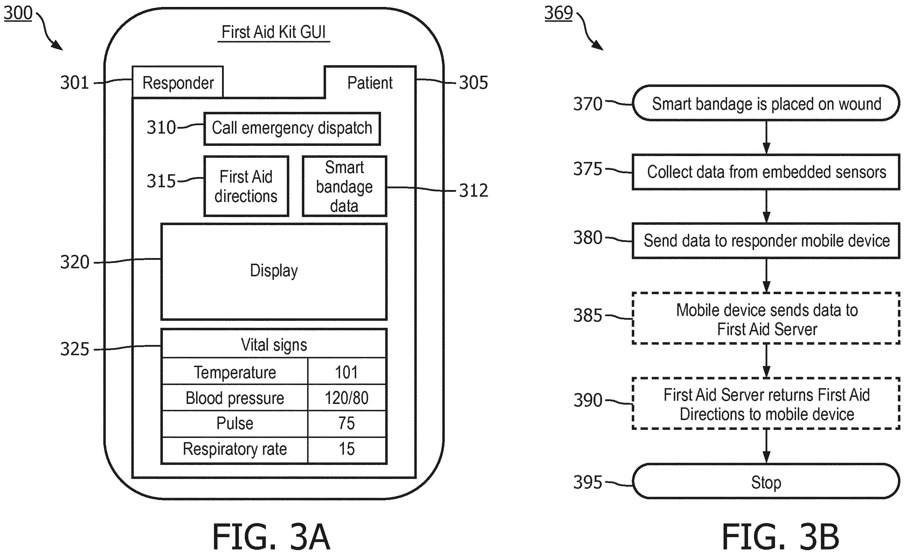

FIGURES 3A-B illustrates an example first aid kit GUI 300 and a flowchart 369 of a smart bandage 182 methodology.

FIGURE 3A illustrates an example first aid kit graphical user interface (GUI) 301 of the mobile device 140 (or, in some embodiments, the wearable device 120). The example first aid kit graphical user interface (GUI) 300 illustrates one embodiment of the first aid kit graphical user interface (GUI) 152, but it should be noted that other embodiments of the first aid kit graphical user interface (GUI) 152 may operate differently.

The example first aid kit GUI 152 includes a responder tab 301 and a patient tab 305, where the responder tab 300 is selected. The example first aid kit GUI 300 may include several selection boxes (*e.g.,* smart bandage data box 312, call emergency dispatch box 310, first aid directions box 315), a display box 320, and a set of vital signs data in a table 325. The selection boxes in the example first aid kit GUI 152 are "call emergency dispatch" 310, "smart bandage data" 312, and "first aid directions" 315. When the "call emergency dispatch" selection box 310 is selected, a call may be placed to 911 or another emergency responder through the mobile device's communication interface 144 or through the wearable device's communication interface 124 or beacon 132. When the "first aid directions" selection box 315 is selected, a set of directions may be displayed in the GUI 152 that may correspond to the set of vital signs (*e.g.,* at the display 320 or alongside the vital signs 325). Directions that are displayed in the GUI 152 may, for example, relate to how to dress a wound. The example vital signs 325 illustrated in the vital signs data table 325 of the first aid GUI 152 include a temperature of 101 degrees F, blood pressure of 120/80, a pulse rate of 75, and a respiratory rate of 15. When the "smart bandage data" selection box 312 is selected, the mobile device may retrieve or display previously received data from the smart bandage 182.

The GUI elements of the example first aid kit GUI 300 of FIGURE 3A should be interpreted illustrative rather than limiting. Another embodiment of the first aid kit GUI 152 may not include all of these GUI elements or may include additional or different GUI elements not listed herein.

FIGURE 3B is a flowchart 369 illustrating example operations of a smart bandage 182. For example, the smart bandage 182 may include a microcontroller or ASIC configured to perform the method of the flowchart 369. The flowchart of the smart bandage methodology begins with step 370 where a smart bandage 182 is placed on a wound. In step 375, data is collected from sensors 184 which may be embedded in the smart bandage 182. Then in step 380 of the smart bandage 182 methodology, data is sent from the sensors 184a,b,c of the smart bandage 182 to a responder mobile device 140, and then the smart bandage sensor data may be sent to a first aid server 160 in step 385, either from the responder mobile device 140 or directly from the smart bandage 182. Finally, in step 390 of the smart bandage 182 method, first aid directions are sent to the responder and those first aid directions may be displayed at the first aid kit GUI 152 at the responder mobile device 140 or, in some embodiments, on a GUI 128 of the responder's wearable device 110. The first aid directions that are displayed at the GUI (152 or 128) may, for example, relate to how to dress a wound with a gauze bandage. The method then proceeds to end in step 395.

While the flow diagram in FIGURE 3B shows a particular order of operations performed by various embodiments, it should be understood that such order is an example (*e.g.,* alternative embodiments may perform the operations in a different order, combine certain operations, overlap certain operations, etc.).

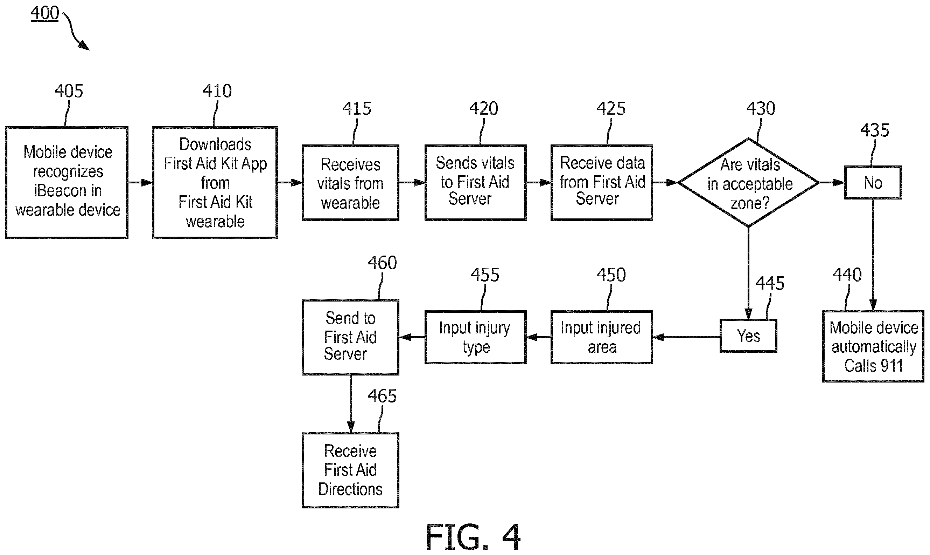

FIGURE 4 is a flowchart 400 illustrating an example methodology that may be performed by base instructions 154 in a mobile device 140 first aid application (App) 150. In step 405 in the method of FIGURE 4, a mobile device may recognize a beacon 132 or communication interface 124 from a wearable device 110. In step 410, the mobile device 140 downloads or installs a first aid kit App 150 from a first aid server 160 or from the wearable device 110 (if it has not already done so). For example, upon pressing a button on the wearable device or upon bringing the mobile device within wireless (*e.g.* NFC or Bluetooth) range of the wearable device, the wearable device may transmit program instructions defining the first aid kit app 150 or a resource identifier (*e.g.,* a URL or app ID known to an application store/marketplace server) indicating from where such instructions may be downloaded. Alternatively, the wearable device or first aid kit may include indicia that may be scanned and recognized by the mobile device (*e.g.,* a QR code, barcode, identifying image, or optically-recognizable letters or other characters to be read by a camera of the mobile device). Such indicia may convey a resource identifier indicating from where such instructions may be downloaded. Next, the method may move to step 415, where vital sign measurement data may be retrieved from the wearable device 110 (*e.g.,* via direct connection 108 or via the data network 101 through connections 102 and 104). Next, in step 420, the vital signs data retrieved is sent to a first aid server 160 by either the mobile device 140 or by the wearable device 110. Then data may be received from the first aid server 160 by the mobile device 140 or the wearable device 110 in step 425. In step 430, the mobile device 140 may determine whether the vital signs are in an acceptable zone. When the vital signs data is determined not to be in an acceptable zone in step 435, the mobile device 140 may automatically call 911 or another emergency responder in step 440. When the vital signs data is determined to be in an acceptable zone in step 445, the program flow of the base instructions 154 moves to step 450, where the base instructions 154 may receive an input relating to an injured area and step 455 where an input may be received relating to an injury type, for example, through first aid kit GUI 152. The injury area and injury type data may then be sent to the first aid server 160 in step 460 and first aid instructions may be received by the mobile device 140 in step 465 and from the first aid server 160.

While the flow diagram in FIGURE 4 shows a particular order of operations performed by various embodiments, it should be understood that such order is an example (*e.g.,* alternative embodiments may perform the operations in a different order, combine certain operations, overlap certain operations, etc.).

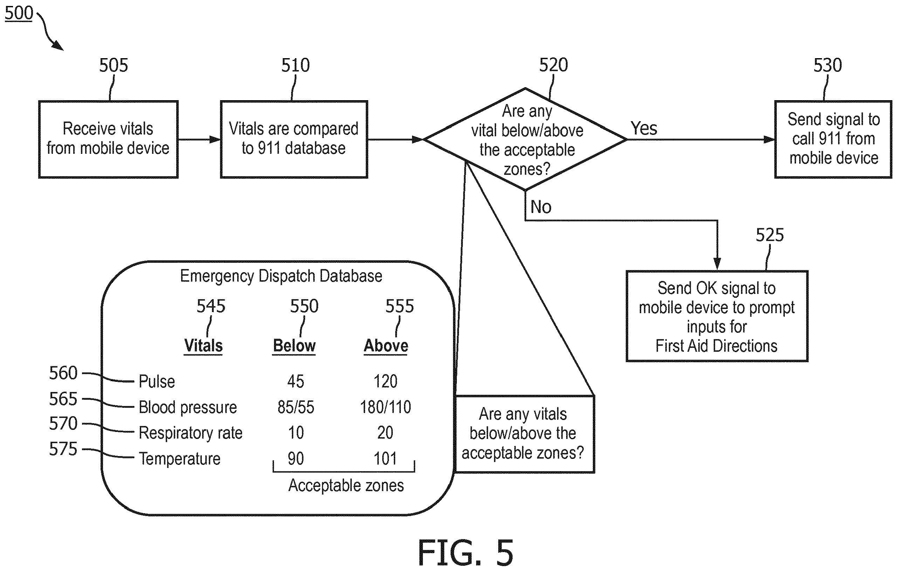

FIGURE 5 is a flowchart 500 illustrating example operations of an emergency dispatch instructions 162 of a first aid server 160. FIGURE 5 also illustrates an example table of vital signs stored in an example emergency dispatch database 164. Data illustrated inside the example emergency dispatch database 164 of FIGURE 5 is intended to be illustrative rather than limiting. The table in the emergency dispatch database 540 identifies acceptable ranges (*e.g.,* vitals 545 should not fall below lower bounds 550 or exceed upper bounds 555) of vital signs data. In step 505 of the example emergency dispatch method of FIGURE 5, vital sign sensor measurements may be received by a first aid server system 160 running emergency dispatch instructions 162 (*e.g.,* from a wearable device 110 or from a mobile device 140 that obtained them from the wearable device 110). Then in step 510, the measured vital signs may be compared to the information in the emergency dispatch database 164. Next, in step 520, the example emergency dispatch instructions 162 determines whether the measured vital signs are within or outside acceptable zones (*e.g.,* lower bound 550, upper bound 555). It will be appreciated that, in various embodiments, the decision 520 may be replaced with alternative decisions (or series' thereof) that are more or less complex than illustrated. For example, in some embodiments, the decision of whether to contact an emergency dispatch in step 530 may be alternatively or additionally based on information regarding the items available in the first aid kit or items removed from or otherwise being used from the kit. For example, in the situation where the vitals 545 or user self-reporting indicates that the user has suffered a laceration that requires immediate dressing, the decision block may direct flow to step 525 if the kit includes gauze and scissors but may direct flow to step 530 if the kit is lacking either of these items (*e.g.,* if the kit was no initially provided with these items or if they have been previously removed and not replaced). In some embodiments, the method 500 may distinguish between more than one level of action to take in response to vitals or other data. For example, depending on the severity or other context of the situation (*e.g.,* as determined by vitals, available first aid kit contents, first aid kit contents selected for use, *etc.*) the method 500 may transmit first aid instructions, contact an emergency public dispatch (*e.g.,* 911 in the US), contact or dispatch first responders directly (bypassing the emergency dispatch), contact a private emergency response organization, establish communication with a private or public hotline for providing first aid guidance, or establish communication with an emergency contact (parent, spouse, physician, *etc.*) as identified by information stored in the user's mobile device. Such examples of the functionality of decision block 520 may be implemented via machine learning. For example, logistic regression may be used to train a classification algorithm on a large data set including example inputs (*e.g.,* vitals, first aid kit contents, first aid items in use, user input, *etc.*) and "correct" actions. Subsequently, the trained algorithm may be used as decision block 520 and, in some embodiments, may receive further training and adjustment in response to real-world scenarios (*e.g.,* using user input as to the user's desired action or as to whether a taken action was correct).

It will be apparent that, while the method 500 is described as being performed by the first aid server, that similar methods for making determinations as to appropriate responses (*e.g.,* to request emergency assistance or request or display first aid instructions) may be

As noted previously, the emergency dispatch instructions may check what the acceptable zones are through the emergency dispatch database 164. When the measured vital signs are outside of an acceptable zone (*e.g.,* the measured vital signs fall below lower bounds 550 or exceed upper bounds 555), the first aid server 160 sends a signal to the mobile device 140 that causes the mobile device to call 911 or another emergency responder in step 530. When the vital signs are within acceptable zones, then the first aid server 160 may send a message to the mobile device 140 indicating that the patient is alright (an "OK signal") in step 525. The first aid server 160 may also send prompts to the mobile device requesting inputs from which first aid directions may be identified in step 525.

The example emergency dispatch database 164 of FIGURE 5 stores four types of vitals - pulse 560, blood pressure 565, respiratory rate 570, and temperature 575. The acceptable zones are each defined by lower bounds 550 and upper bounds 555. An acceptable pulse 560, according to the example emergency dispatch database 164 of FIGURE 5, should be between 45 and 120. Similarly, an acceptable blood pressure 565 should be between 85/55 and 180/110; an acceptable respiratory rate 570 should be between 10 and 20; and an acceptable temperature 575 should be between 90 and 101. The example emergency dispatch database 164 of FIGURE 5 should be interpreted as illustrative rather than limiting, and may describe acceptable zones for other vital signs, or may be missing vital signs shown in FIGURE 5.

While the flow diagram in FIGURE 5 shows a particular order of operations performed by various embodiments, it should be understood that such order is an example (*e.g.,* alternative embodiments may perform the operations in a different order, combine certain operations, overlap certain operations, etc.).

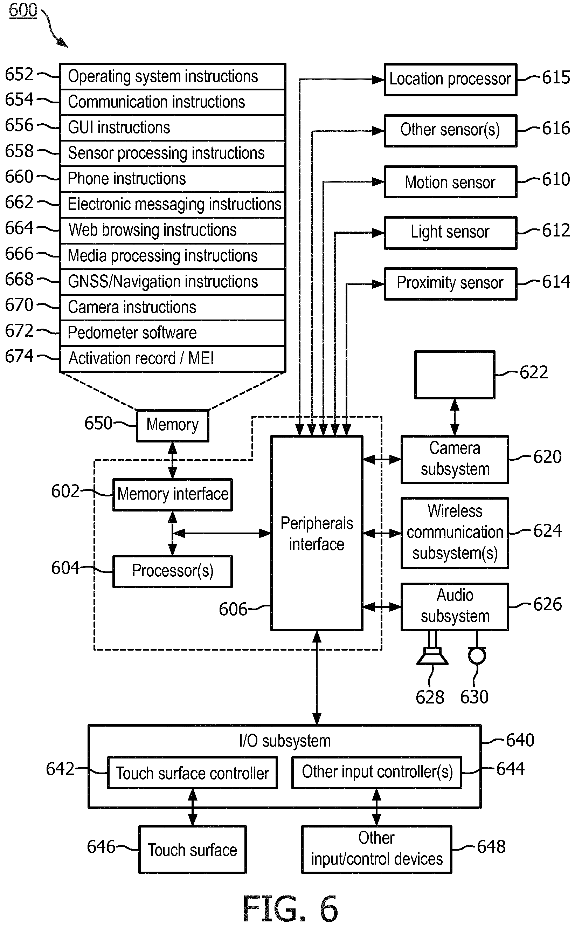

FIGURE 6 illustrates a mobile device architecture that may be utilized to implement the various features and processes described herein. Architecture 600 may be implemented in any number of portable devices including but not limited to smart wearable devices. Architecture 600 as illustrated in FIGURE 6 includes memory interface 602, processors 604, and peripheral interface 606. Memory interface 602, processors 604 and peripherals interface 606 may be separate components or may be integrated as a part of one or more integrated circuits. The various components may be coupled by one or more communication buses or signal lines.

Processors 604 as illustrated in FIGURE 6 are meant to be inclusive of data processors, image processors, central processing unit, or any variety of multi-core processing devices. Any variety of sensors, external devices, and external subsystems may be coupled to peripherals interface 606 to facilitate any number of functionalities within the architecture 600 of the exemplar mobile device. For example, motion sensor 610, light sensor 612, and proximity sensor 614 may be coupled to peripherals interface 606 to facilitate orientation, lighting, and proximity functions of the mobile device. For example, light sensor 612 may be utilized to facilitate adjusting the brightness of touch surface 646. Motion sensor 610, which may be exemplified in the context of an accelerometer or gyroscope, may be utilized to detect movement and orientation of the mobile device. Display objects or media may then be presented according to a detected orientation (*e.g.,* portrait or landscape).

Other sensors may be coupled to peripherals interface 606, such as a temperature sensor, a heart rate sensor, other vitals sensors, or other sensing device to facilitate corresponding functionalities. Location processor 615 (*e.g.,* a global positioning transceiver) may be coupled to peripherals interface 606 to allow for generation of geo-location data thereby facilitating geo-positioning. An electronic magnetometer 616 such as an integrated circuit chip may in turn be connected to peripherals interface 606 to provide data related to the direction of true magnetic North whereby the mobile device may enjoy compass or directional functionality. Camera subsystem 620 and an optical sensor 622 such as a charged coupled device (CCD) or a complementary metal-oxide semiconductor (CMOS) optical sensor may facilitate camera functions such as recording photographs and video clips.

Communication functionality may be facilitated through one or more communication subsystems 624, which may include one or more wireless communication subsystems. Wireless communication subsystems 624 may include 802.5 or Bluetooth transceivers as well as optical transceivers such as infrared. Wired communication system may include a port device such as a Universal Serial Bus (USB) port or some other wired port connection that may be used to establish a wired coupling to other computing devices such as network access devices, personal computers, printers, displays, or other processing devices capable of receiving or transmitting data. The specific design and implementation of communication subsystem 624 may depend on the communication network or medium over which the device is intended to operate. For example, a device may include wireless communication subsystem designed to operate over a global system for mobile communications (GSM) network, a GPRS network, an enhanced data GSM environment (EDGE) network, 802.5 communication networks, code division multiple access (CDMA) networks, or Bluetooth networks. Communication subsystem 624 may include hosting protocols such that the device may be configured as a base station for other wireless devices. Communication subsystems may also allow the device to synchronize with a host device using one or more protocols such as TCP/IP, HTTP, or UDP.

Audio subsystem 626 may be coupled to a speaker 628 and one or more microphones 630 to facilitate voice-enabled functions. These functions might include voice recognition, voice replication, or digital recording. Audio subsystem 626 in conjunction may also encompass traditional telephony functions.

I/O subsystem 640 may include touch controller 642 or other input controller(s) 644. Touch controller 642 may be coupled to a touch surface 646. Touch surface 646 and touch controller 642 may detect contact and movement or break thereof using any of a number of touch sensitivity technologies, including but not limited to capacitive, resistive, infrared, or surface acoustic wave technologies. Other proximity sensor arrays or elements for determining one or more points of contact with touch surface 646 may likewise be utilized. In one implementation, touch surface 646 may display virtual or soft buttons and a virtual keyboard, which may be used as an input/output device by the user.

Other input controllers 644 may be coupled to other input/control devices 648 such as one or more buttons, rocker switches, thumb-wheels, infrared ports, USB ports, or a pointer device such as a stylus. The one or more buttons (not shown) may include an up/down button for volume control of speaker 628 or microphone 630. In some implementations, device 600 may include the functionality of an audio or video playback or recording device and may include a pin connector for tethering to other devices.

Memory interface 602 may be coupled to memory 650. Memory 650 may include high-speed random access memory or non-volatile memory such as magnetic disk storage devices, optical storage devices, or flash memory. Memory 650 may store operating system 652, such as Darwin, RTXC, LINUX, UNIX, OS X, ANDROID, WINDOWS, or an embedded operating system such as VXWorks. Operating system 652 may include instructions for handling basic system services and for performing hardware dependent tasks. In some implementations, operating system 652 may include a kernel.

Memory 650 may also store communication instructions 654 to facilitate communicating with other mobile computing devices or servers. Communication instructions 654 may also be used to select an operational mode or communication medium for use by the device based on a geographic location, which may be obtained by the GPS/Navigation instructions 668. Memory 650 may include graphical user interface instructions 656 to facilitate graphic user interface processing such as the generation of an interface; sensor processing instructions 658 to facilitate sensor-related processing and functions; phone instructions 660 to facilitate phone-related processes and functions; electronic messaging instructions 662 to facilitate electronic-messaging related processes and functions; web browsing instructions 664 to facilitate web browsing-related processes and functions; media processing instructions 666 to facilitate media processing-related processes and functions; GPS/Navigation instructions 668 to facilitate GPS and navigation-related processes, camera instructions 670 to facilitate camera-related processes and functions; pedometer software 672 to facilitate pedometer-related process; activation record/IMEI software 674 to facilitate activation record/IMEI-related processes; and instructions for any other application that may be operating on or in conjunction with the mobile computing device. Memory 650 may also store other software instructions for facilitating other processes, features and applications, such as applications related to navigation, social networking, location-based services or map displays.

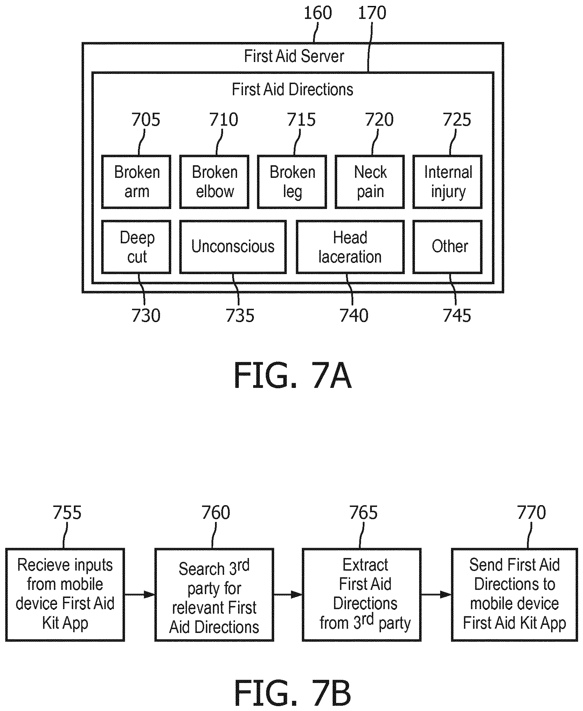

FIGURE 7A is a diagram of example injury-type selection boxes relating to first aid directions 170 that may be selected at an example first aid kit GUI 152 of the mobile device 140. First aid selection boxes presented by the example first aid kit GUI 152 of FIGURE 7A include broken arm 705, broken elbow 710, broken leg 720, pain from neck 720, internal injury 725, deep cut 730, unconscious 735, head laceration 740, and other injuries 745. An individual seeking first aid instructions may simply select an appropriate selection box to receive relevant instructions at his/her mobile device 140 or wearable device 110 from the first aid server 160. For example, when broken arm 705 is selected at the first aid kit GUI 152, first aid instructions may sent to a user mobile device 140 recommending that the arm not be moved, and to apply ice to the injury.

FIGURE 7B is a flowchart illustrating an example process of generating first aid directions 170 at a base instructions 154 of the first aid server 160. In some embodiments, this example process may be executed by a processor running on a third party source 174. In step 755, inputs may be received by the first aid server 160 from a first aid kit application 150 of a mobile device 140. After the inputs have been received the first aid server 160, the first aid server 160 may search a third party source 174 (*e.g.,,* a third party server or a third party database) for relevant first aid directions in step 760. Next, in step 765, the relevant first aid directions may be extracted from the third party source 174, and, in some embodiments, stored at the first aid server 160. Then, in step 770 of the method of FIGURE 7B, the first aid directions are sent to the first aid kit application 150 of the mobile device 140, and in some embodiments, also to the wearable device 110.

While the flow diagrams in FIGURE 7A and FIGURE 7B show a particular order of operations performed by various embodiments, it should be understood that such order is an example (*e.g.,* alternative embodiments may perform the operations in a different order, combine certain operations, overlap certain operations, etc.).

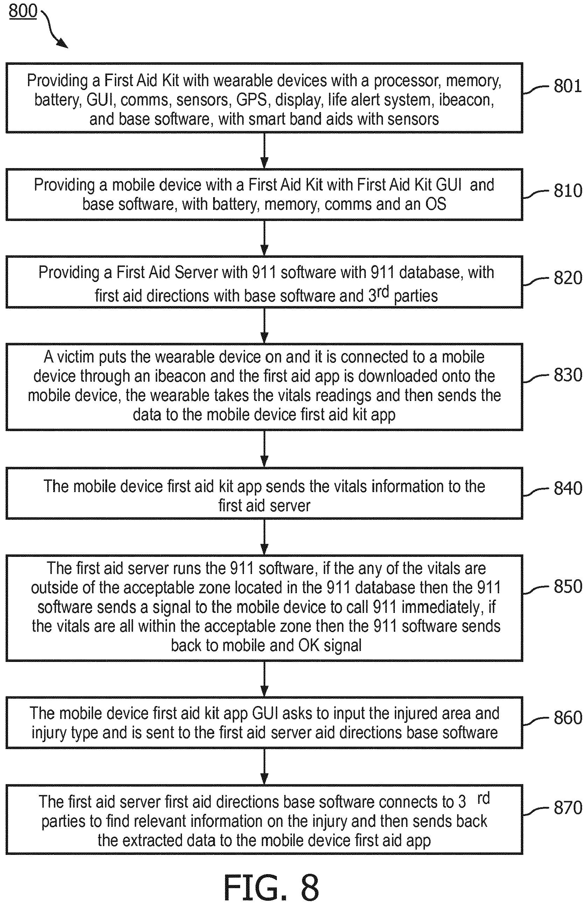

FIGURE 8 is a flowchart 800 illustrating an example methodology consistent with the example methods and principles described herein. FIGURE 8 begins with step 801, which includes providing a wearable device 110 with a processor 112, a memory 114, a battery 118, a GUI 128, a communication interface (comms) 124, a display 120, an alert instructions 130, a beacon 132, a base instructions 126, and sensors 116, which may include band aid sensors 182 associated with the wearable device 110. Then, in step 810 of the method, a mobile device 140 is provided with a first aid kit application 150, with a first aid kit GUI 152, with base instructions 154, with a battery 146, with a memory 142, with a communications interface 144, and with an operating system 148. Next, a first aid server 160 is provided with emergency dispatch instructions 162, a emergency dispatch database 164, a first aid directions database 170, with first aid directions base instructions 172, and with communication interfaces that may be used to communicate with third party sources in step 820.

In step 830 of the method of FIGURE 8, a victim/patient/user puts on a wearable device 110, the wearable device 110 connects to the mobile device 140 using a beacon 132 or communication interface 124, a first aid application 150 is downloaded onto the mobile device 140, the wearable device measures vital signs with sensors 116, and then the wearable device sends the vital sign sensor measurement data to the first aid kit app 150 of the mobile device 140.

In step 840 of the method of FIGURE 8, the mobile device 140 first aid kit application 150 sends the vital sign measurement data to a first aid server 160. Then, in step 850, the first aid server 160, running emergency dispatch instructions 162, identifies whether any vital sign is outside of an acceptable zone. When a vital sign is in an unacceptable level the emergency dispatch instructions may send a signal to the mobile device that causes the first aid kit instructions running on the mobile device to initiate a call to emergency dispatch in step 850. When all vital signs are within an acceptable zone the first aid server sends a message to the mobile device indicating that the person wearing the wearable device is OK in step 850.

In step 860 of the method of FIGURE 8, the mobile device 140 first aid kit application GUI 152 queries a user of the mobile device to enter an injury area and an injury type (*e.g.,* first aid direction selection boxes of FIGURE 7A). After the injury area and injury type are sent entered into the GUI, they may be sent to the first aid server 160 in step 860. Finally, in step 870 of FIGURE 8, the first aid server 160 queries third parties 174 for relevant first aid response directions 170 and the first aid server 160 forwards those directions to the mobile device 140, or, in some embodiments, the wearable device 110.

While the flow diagram in FIGURE 8 shows a particular order of operations performed by various embodiments, it should be understood that such order is an example (*e.g.,* alternative embodiments may perform the operations in a different order, combine certain operations, overlap certain operations, etc.).

According to the foregoing, various embodiments enable the provision of a low-cost wearable device within a first aid kit. For example, by providing a first aid kit that automatically or otherwise easily loads instructions onto a user's mobile device for communicating with and interpreting data from a wearable device, functions normally performed by a wearable device may be moved to the mobile device. As such, processing power and other resources required by the wearable device are reduced, leading to a lower cost wearable device that can be mass-produced for inclusion in similarly-low cost first aid kits.

Further, inclusion of a wearable device in the first aid kit enables for enhanced assistance when a first aid kit is accessed. While first aid kits offer a portable hospital supply, in a sense, they do not provide similar down-scaled patient monitoring solutions which may be valuable in an emergency situation. By providing a wearable within the first aid kit, such monitoring may be achieved in the field so that critical information can be observed by the first-aid kit user and relayed to emergency medical personnel, for example, via the phone. Various additional benefits and technical advantages will be apparent in view of the foregoing.

It should be apparent from the foregoing description that various example embodiments of the invention may be implemented in hardware or firmware. Furthermore, various example embodiments may be implemented as instructions stored on a machine-readable storage medium, which may be read and executed by at least one processor to perform the operations described in detail herein. A machine-readable storage medium may include any mechanism for storing information in a form readable by a machine, such as a personal or laptop computer, a server, or other computing device. Thus, a machine-readable storage medium may include read-only memory (ROM), random-access memory (RAM), magnetic disk storage media, optical storage media, flash-memory devices, and similar storage media.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in machine readable media and so executed by a computer or processor, whether or not such computer or processor is explicitly shown.

Although the various example embodiments have been described in detail with particular reference to certain example aspects thereof, it should be understood that the invention is capable of other embodiments and its details are capable of modifications in various obvious respects. As is readily apparent to those skilled in the art, variations and modifications may be affected while remaining within the scope of the invention. Accordingly, the foregoing disclosure, description, and figures are for illustrative purposes only and do not in any way limit the invention, which is defined only by the claims.

## Claims

1. A first aid kit (180) comprising:
at least one first aid item (190-199) including a first first-aid item associated with an identifier; and
a wearable device (110) configured to be worn by a user and comprising:
a communications interface (124) for communicating with a mobile device (140) of the user;
a sensor (116) for obtaining vitals data when the wearable device is worn by the user;
a processor (112) configured to:
transmit vitals data to a mobile device of the user via the communications interface,
read the identifier from the first first-aid item, and (a) determine, based at least in part of reading the identifier, that the first first-aid item is in use, and transmit, to the mobile device, an indication that first first-aid item is in use; or (b) transmit the identifier to the mobile device via the communication interface.

2. The first aid kit of claim 1, wherein the first aid kit comprises means for causing instructions for processing the vitals data to be loaded onto the mobile device, comprising:
a memory storing contents comprising at least one of:
the instructions for processing the vitals data, and
a resource identifier that points to the instructions for processing the vitals data at an application download server,
wherein the contents of the memory are transmitted to the mobile device.

3. The first aid kit of claim 2, wherein the wearable device comprises the memory.

4. The first aid kit of claim 1, wherein the first aid kit comprises means for causing instructions for processing the vitals data to be loaded onto the mobile device, comprising:
a visible indicia that, when read by a camera of the mobile device, communicates a resource identifier that points to the instructions for processing the vitals data at an application download server.

5. The first aid kit of claim 1, wherein, in reading the identifier, the processor is configured to read the identifier via short-range wireless communication.

6. The first aid kit of any of claims 1-5, wherein the processor is further configured to transmit to the mobile device an identification of contents within the first-aid kit.

7. A wearable device (110) for inclusion in a first aid kit (180) with at least one first aid kit item, (190-199) the wearable device comprising:
a communications interface (124) for communicating with a mobile device (140) of a user;
a sensor (116) for obtaining vitals data when the wearable device is worn by the user;
a processor (112) configured to:
transmit vitals data to a mobile device of the user via the communications interface;
read an identifier from a first first-aid item of the at least one first aid kit item, and (a) determine, based at least in part of reading the identifier, that the at least one first first-aid item is in use; and transmit, to the mobile device, an indication that the first first-aid item is in use; or (b) transmit the identifier to the mobile device via the communication interface.

8. The wearable device of claim 7, further comprising:
a memory storing contents comprising at least one of:
the instructions for processing the vitals data, and
a resource identifier that points to the instructions for processing the vitals data at an application download server,
wherein the processor is further configure to transmit the contents of the memory to the mobile device.

9. The wearable device of claim 7, further comprising:
a visible indicia that, when read by a camera of the mobile device, communicates a resource identifier that points to the instructions for processing the vitals data at an application download server.

10. The wearable device of claim 7, wherein, in reading the identifier, the processor is configured to read the identifier via short-range wireless communication.

11. The wearable device of any of claims 7-10, wherein the processor is further configured to transmit to the mobile device an identification of contents within the first-aid kit.

## Patentansprüche

1. Erste-Hilfe-Set (180), das Folgendes umfasst:
mindestens ein Erste-Hilfe-Element (190 bis 199),
das ein Erste-Hilfe-Element beinhaltet, das mit einem Identifikator assoziiert ist; und
eine tragbare Vorrichtung (110), die konfiguriert ist, um von einem Benutzer getragen zu werden, und die Folgendes umfasst:
eine Kommunikationsschnittstelle (124)
zur Kommunikation mit einem Mobilgerät (140) des Benutzers;
einen Sensor (116) zum Erhalten von Vitaldaten, wenn die tragbare Vorrichtung von dem Benutzer getragen wird;
einen Prozessor (112), der konfiguriert ist, um:
Vitaldaten zu einem Mobilgerät des Benutzers über die Kommunikationsschnittstelle zu übertragen,
den Identifikator aus dem Erste-Hilfe-Element zu lesen und (a), mindestens zum Teil basierend auf dem Lesen des Identifikators, zu bestimmen, dass das Erste-Hilfe-Element in Verwendung ist, und zu dem Mobilgerät eine Angabe zu übertragen, dass das Erste-Hilfe-Element in Verwendung ist; oder (b) den Identifikator zu dem Mobilgerät über die Kommunikationsschnittstelle zu übertragen.

2. Erste-Hilfe-Set nach Anspruch 1, wobei das Erste-Hilfe-Set Mittel umfasst, um zu veranlassen, dass Anweisungen zum Verarbeiten der Vitaldaten auf das Mobilgerät geladen werden, das Folgendes umfasst: einen Speicher, der Inhalt speichert, der mindestens eines der folgenden umfasst:
die Anweisungen zum Verarbeiten der Vitaldaten, und
ein Ressourcenidentifikator, der zu den Anweisungen zum Verarbeiten der Vitaldaten an einem Anwendungsdownloadserver zeigt,
wobei der Inhalt des Speichers zu dem Mobilgerät übertragen wird.

3. Erste-Hilfe-Set nach Anspruch 2, wobei die tragbare Vorrichtung den Speicher umfasst.

4. Erste-Hilfe-Set nach Anspruch 1, wobei das Erste-Hilfe-Set Mittel umfasst, um zu veranlassen, dass Anweisungen zum Verarbeiten der Vitaldaten auf das Mobilgerät geladen werden, das Folgendes umfasst:
einen sichtbaren Hinweis, der, wenn er von einer Kamera des Mobilgeräts gelesen wird, einen Ressourcenidentifikator mitteilt, der zu den Anweisungen zum Verarbeiten der Vitaldaten an einem Anwendungsdownloadserver zeigt.

5. Erste-Hilfe-Set nach Anspruch 1, wobei der Prozessor konfiguriert ist, um den Identifikator über drahtlose Kurzstreckenkommunikation zu lesen.

6. Erste-Hilfe-Set nach einem der Ansprüche 1 bis 5, wobei der Prozessor weiter konfiguriert ist, um zu dem Mobilgerät eine Identifikation des Inhalts innerhalb des Erste-Hilfe-Sets zu übertragen.

7. Tragbare Vorrichtung (110), die zu einem Erste-Hilfe-Set (180) gehört, mit mindestens einem ersten Erste-Hilfe-Set-Element (190 bis 199), wobei die tragbare Vorrichtung Folgendes umfasst:
eine Kommunikationsschnittstelle (124) zur Kommunikation mit einem Mobilgerät (140) eines Benutzers;
einen Sensor (116) zum Erhalten von Vitaldaten, wenn die tragbare Vorrichtung von dem Benutzer getragen wird;
einen Prozessor (112), der konfiguriert ist, um:
Vitaldaten zu einem Mobilgerät des Benutzers über die Kommunikationsschnittstelle zu übertragen;
einen Identifikator aus einem ersten Erste-Hilfe-Element des mindestens einen ersten Erste-Hilfe-Set-Elements zu lesen und (a), mindestens zum Teil basierend auf dem Lesen des Identifikators zu bestimmen, dass das mindestens eine erste Erste-Hilfe-Element in Verwendung ist; und zu dem Mobilgerät eine Angabe zu übertragen, dass das erste Erste-Hilfe-Element in Verwendung ist; oder (b) den Identifikator zu dem Mobilgerät über die Kommunikationsschnittstelle zu übertragen.

8. Tragbare Vorrichtung nach Anspruch 7, die weiter Folgendes umfasst:
einen Speicher, der Inhalte speichert, die mindestens eines der folgenden umfassen:
die Anweisungen zum Verarbeiten der Vitaldaten, und
einen Ressourcenidentifikator, der zu den Anweisungen zum Verarbeiten der Vitaldaten an einem Anwendungsdownloadserver zeigt,
wobei der Prozessor weiter konfiguriert ist, um den Inhalt des Speichers zu dem Mobilgerät zu übertragen.

9. Tragbare Vorrichtung nach Anspruch 7, die weiter Folgendes umfasst:
einen sichtbaren Hinweis, der, wenn er von einer Kamera des Mobilgeräts gelesen wird, einen Ressourcenidentifikator mitteilt, der zu den Anweisungen zum Verarbeiten der Vitaldaten an einem Anwendungsdownloadserver zeigt.

10. Tragbare Vorrichtung nach Anspruch 7, wobei der Prozessor konfiguriert ist, um den Identifikator über drahtlose Kurzstreckenkommunikation zu lesen.

11. Tragbare Vorrichtung nach einem der Ansprüche 7 bis 10, wobei der Prozessor weiter konfiguriert ist, um zu dem Mobilgerät eine Identifikation des Inhalts innerhalb des Erste-Hilfe-Sets zu übertragen.

## Revendications

1. Kit de premiers soins (180) comprenant :
au moins un article de premiers soins (190-199) comprenant un premier article de premiers soins associé à un identifiant ; et
un dispositif portable (110) configuré pour être porté par un utilisateur et comprenant :
une interface de communications (124) pour communiquer avec un dispositif mobile (140) de l'utilisateur ;
un capteur (116) pour obtenir des données vitales lorsque le dispositif portable est porté par l'utilisateur ;
un processeur (112) configuré pour :
transmettre des données vitales à un dispositif mobile de l'utilisateur via l'interface de communications,
lire l'identifiant dans le premier article de premiers soins et (a) déterminer, sur la base au moins en partie de la lecture de l'identifiant, que le premier article de premiers soins est en service et transmettre au dispositif mobile une indication de ce que le premier article de premiers soins est en service ; ou (b) transmettre l'identifiant au dispositif mobile via l'interface de communications.

2. Kit de premiers soins selon la revendication 1, dans lequel le kit de premiers soins comprend des moyens pour amener le chargement d'instructions de traitement des données vitales sur le dispositif mobile, comprenant :
une mémoire stockant des contenus comprenant au moins l'un des suivants :
les instructions pour le traitement des données vitales et
un identifiant de ressource qui pointe les instructions pour le traitement des données vitales dans un serveur de téléchargement d'application,
dans lequel les contenus de la mémoire sont transmis au dispositif mobile.

3. Kit de premiers soins selon la revendication 2, dans lequel le dispositif portable comprend la mémoire.

4. Kit de premiers soins selon la revendication 1, dans lequel le kit de premiers soins comprend des moyens pour amener le chargement d'instructions pour le traitement des données vitales sur le dispositif mobile, comprenant :
un repère visible qui, lorsqu'il est lu par une caméra du dispositif mobile, communique un identifiant de ressource qui pointe les instructions de traitement des données vitales dans un serveur de téléchargement de l'application.

5. Kit de premiers soins selon la revendication 1, dans lequel, lors de la lecture de l'identifiant, le processeur est configuré pour lire l'identifiant via une communication sans fil à courte portée.

6. Kit de premiers soins selon l'une quelconque des revendications 1-5, dans lequel le processeur est en outre configuré pour transmettre au dispositif mobile une identification de contenus dans le kit de premiers soins.

7. Dispositif portable (110) pour inclusion dans un kit de premiers soins (180) avec au moins un article de kit de premiers soins (190-199), le dispositif portable comprenant :
une interface de communications (124) pour communiquer avec un dispositif mobile (140) d'un utilisateur ;
un capteur (116) pour obtenir des données vitales lorsque le dispositif portable est porté par l'utilisateur ;
un processeur (112) configuré pour :
transmettre des données vitales à un dispositif mobile de l'utilisateur via l'interface de communications,
lire l'identifiant dans le premier article de premiers soins et (a) déterminer, sur la base au moins en partie de la lecture de l'identifiant, que le premier article de premiers soins est en service et transmettre au dispositif mobile une indication de ce que le premier article de premiers soins est en service ; ou (b) transmettre l'identifiant au dispositif mobile via l'interface de communications.

8. Dispositif portable selon la revendication 7, comprenant en outre :
une mémoire stockant des contenus comprenant au moins l'un des suivants :
les instructions pour le traitement des données vitales et
un identifiant de ressource qui pointe les instructions pour le traitement des données vitales dans un serveur de téléchargement d'application,
dans lequel le processeur est en outre configuré pour transmettre les contenus de la mémoire au dispositif mobile.

9. Dispositif portable selon la revendication 7, comprenant en outre :
un repère visible qui, lorsqu'il est lu par une caméra du dispositif mobile, communique un identifiant de ressource qui pointe les instructions de traitement des données vitales dans un serveur de téléchargement de l'application.

10. Dispositif portable selon la revendication 7, dans lequel, lors de la lecture de l'identifiant, le processeur est configuré pour lire l'identifiant via une communication sans fil à courte portée.

11. Dispositif portable selon l'une quelconque des revendications 7-10, dans lequel le processeur est en outre configuré pour transmettre au dispositif mobile une identification de contenus dans le kit de premiers soins.
